(19) Europäisches Patentamt
European Patent Office
Office européen des brevets

(11) EP 4 197 525 A1

(12) **EUROPEAN PATENT APPLICATION**

(43) Date of publication:
**21.06.2023 Bulletin 2023/25**

(21) Application number: **21216181.4**

(22) Date of filing: **20.12.2021**

(51) International Patent Classification (IPC):
***A61K 9/00*** *(2006.01)* ***A61K 31/41*** *(2006.01)*
***A61K 31/4745*** *(2006.01)* ***A61K 39/00*** *(2006.01)*
***A61K 45/06*** *(2006.01)* ***A61P 35/00*** *(2006.01)*
***A61P 35/04*** *(2006.01)*

(52) Cooperative Patent Classification (CPC):
(C-Sets available)
**A61K 31/41; A61K 9/0019; A61K 9/2004; A61K 9/4841; A61K 31/4745; A61K 39/00; A61K 45/06; A61P 35/00; A61P 35/04** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Scandion Oncology A/S**
**2100 Copenhagen Ø (DK)**

(72) Inventors:
- **JENSEN, Mads, Aabo**
  **2100 Copenhagen Ø (DK)**
- **STENVANG, Jan**
  **2100 Copenhagen Ø (DK)**

(74) Representative: **Birkeland, Morten**
**IPTector Consulting Aps**
**Diplomvej 377, 222**
**2800 Kgs. Lyngby (DK)**

Remarks:
The complete document including Reference Table(s) and the Sequence Listing(s) can be downloaded from the EPO website

(54) **COMBINATION OF DRUGS FOR THE TREATMENT OF CANCER**

(57) The present invention relates to methods for treating a cancer comprising administering to a subject in need thereof an effective amount of a (i) first anti-cancer agent, (ii) an agent sensitizing and/or potentiating the cancer to the first anti-cancer agent, and (iii) a second anti-cancer agent which comprise a PD-1 and/or PD-L1 inhibitor.

EP 4 197 525 A1

(52) Cooperative Patent Classification (CPC): (Cont.)

C-Sets
**A61K 31/41, A61K 2300/00;**
**A61K 31/4745, A61K 2300/00**

## Description

### Technical Field

The present invention relates to treatment for cancer, in particular to methods for sensitising cancer cells to an anti-cancer treatment by administering an effective amount of an agent sensitizing and/or potentiating the cancer to a first anti-cancer agent, and a second anti-cancer agent which comprise a PD-1 and/or PD-L1 inhibitor.

### Background

Cancer is a great burden to our society and there were an estimated 18 million cancer cases around the world in 2018, of these 9.5 million cases were in men and 8.5 million in women. Despite the introduction of many new treatment modalities/options, de novo or acquired resistance to the applied treatments still represents the major cause of death from cancer. Thus, the resistance of cancer cells to anti-cancer drugs is a major reason for the failure of traditional cancer treatment.

A variety of chemo-sensitizing agents has been developed and several have been shown to be effective in experimental models *in vitro* and *in vivo.* These sensitizing agents include, among many others, tyrosine kinase inhibitors, anti-ABC transporters, angiogenesis inhibitors and modulators and/or inhibitors of volume regulated anion channels (VRACs).

However, one problem with receiving chemotherapy is possibility of developing neutropenia. Neutropenia is caused by an abnormally low concentration of neutrophils in the blood. Neutrophils make up the majority of circulating white blood cells and serve as the primary defense against infections by destroying bacteria, bacterial fragments, and immunoglobulin-bound viruses in the blood. Patients with neutropenia are more susceptible to bacterial infections and, the condition may become life-threatening.

PD-1 inhibitors and PD-L1 inhibitors are a group of checkpoint inhibitor anti-cancer drugs that block the activity of PD-1 and PDL1 immune checkpoint proteins present on the surface of cells. PD-1 and PD-L1 inhibitors function to inhibit the association of the programmed death-ligand 1 (PD-L1) with its receptor, programmed cell death protein 1 (PD-1). The interaction of these cell surface proteins is involved in the suppression of the immune system and occurs following infection to limit the killing of bystander host cells and prevent autoimmune disease. Immune checkpoint inhibitors (ICIs) have revolutionized cancer therapy. However, it is estimated that up to 60-70% of patients do not respond to single-agent PD-1 or PD-L1 inhibitor therapy. To address this clinical challenge, different conventional cancer treatment modalities have been tested in combination with ICIs to achieve synergetic effects and to overcome the resistance to immunotherapy. Although some of these approaches have provided clinical benefits, the lack of knowledge in the functional interactions between conventional cancer therapies and immune checkpoint blockades remains a crucial hurdle in developing rational and optimal combination strategies (see for example Ref 1).

WO17198700 disclose a method of sensitising cancer cells to an anti-cancer treatment by administering to a subject in need thereof an effective amount of a VRAC modulator such as SCO-101

Thus, characterisation and clinically validation of new drug combinations and treatment regims for treatment of cancer and in particular drug resistant cancers constitute a highly unmet medical need.

### Summary

Over this background art the present invention provides treatment regimes for improving the anti-cancer effects of conventional anti-cancer agents in combination with a PD-1 and/or PD-L1 inhibitor by administering an effective amount of an agent sensitizing and/or potentiating the cancer to the conventional anti-cancer agents.

As described in working example 1 herein, the inventors surprinsingly and unexpectedly found, in an animal model system, that co-administering an effective amount of an agent, such as SCO-101, sensitizing and/or potentiating the cancer, to an anti-cancer agent such as Irinotecan and/or 5-FU, in combination with an anti-PD-1 antibody, is much more effective than just treatment with the the anti-cancer agent and the anti-PD-1 antibody alone, or the treatment with the the anti-cancer agent and the sensitizing and/or potentiating agent alone.

Accordingly, in a first aspect the present invention provides a method for treating a cancer comprising administering to a subject in need thereof an effective amount of a (i) first anti-cancer agent, (ii) an agent sensitizing and/or potentiating the cancer to the first anti-cancer agent, and (iii) a second anti-cancer agent which comprise a PD-1 and/or PD-L1 inhibitor.

In a second aspect the present invention relates to a pharmaceutical composition comprising the sensitizing/potentiating agent, the first anti-cancer agent, the second anti-cancer agent, and one or more pharmaceutically acceptable adjuvants, excipients, carriers, buffers and/or diluents.

In a third aspect the present invention relates to a composition comprising an effective amount of the sensitizing/potentiating agent, the first anti-cancer agent, and the second anti-cancer agent for use as a medicament.

In a fourth aspect the present invention relates to a composition comprising an effective amount of the sensitizing/potentiating agent, the first anti-cancer agent, and the second anti-cancer agent for use in the treatment of cancer.

In a fifth aspect the present invention relates to a kit of parts comprising (i) a sensitizing/potentiating agent, (ii) a first anti-cancer agent and (iii) a second anti-cancer agent, wherein the second anti-cancer agent comprises a PD-1 and/or PD-L1 inhibitor and the sensitizing/potentiating agent, the first and the second anti-cancer agents are formulated for simultaneous, sequential, or separate administration and optionally instructions for use.

Accordingly, and without being limited by theory, the improved positive effect of administering a sensitizing/potentiating agent, such as SCO-101, together with a combination of a first anti-cancer agent, such as Irinotecan or 5-FU and a second anti-cancer agent comprising anti-PD-1/PD-L1 antibody may be mediated by sensitizing/potentiating agent altering the functionality of the first anti-cancer agent.

## Description of drawings and figures

The figures included herein are illustrative and simplified for clarity, and they merely show details which are essential to the understanding of the invention, while other details may have been left out. When using reference numerals in drawings, throughout the specification, claims and drawings the same reference numerals are used for identical or corresponding parts. The figures and drawing include:

Figure 1 is a schematic representation of an animal survival study showing the anti-tumor activity of SCO-101 combined with a first anti-cancer agent and a second anti-cancer agent which comprise a PD-1 and/or PD-L1 inhibitor targeting antibody in mouse colon tumor model.

## Incorporation by reference

All publications, patents, and patent applications referred to herein are incorporated by reference to the same extent as if each individual publication, patent, or patent application was specifically and individually indicated to be incorporated by reference. In the event of a conflict between a term herein and a term in an incorporated reference, the term herein prevails and controls.

## Detailed description

The features and advantages of the present invention is readily apparent to a person skilled in the art by the below detailed description of embodiments and examples of the invention with reference to the figures and drawings included herein.

## Agent sensitizing and/or potentiating the cancer to the first anti-cancer agent

A variety of chemo-sensitizing agents has been developed and several have been shown to be effective in experimental models *in vitro* and *in vivo.* These sensitizing agents include, among many others, UGT1A1 inhibitors, SRPK1 inhibitors, tyrosine kinase inhibitors, anti-ABC transporter inhibitors, such as ABCG2 inhibitors, angiogenesis inhibitors and modulators and/or inhibitors of volume regulated anion channels (VRACs).

ABCG2 also known as breast cancer resistance protein (bcrp) and is a member of the adenosine triphosphate (ATP)-binding cassette (ABC) family of transporters. ABCG2 is further known to confer resistance to a wide range of chemotherapeutic agents including mitoxantrone, topotecan and irinotecan. ABCG2 is highly expressed in normal and cancer stem cells and has also been detected in a wide variety of untreated human solid tumors.

Volume regulated anion channels (VRAC) are present in most mammalian cells. An important function of VRAC is cell volume regulation: Upon swelling of the cell in hypotonic solution these channels are activated, and chloride ions flow out of the cell in parallel with potassium ions (via potassium channels) and water, thereby restoring the original cell volume.

In one embodiment, the agent sensitizing and/or potentiating the cancer to the first anti-cancer agent is a VRAC blocker. The term "VRAC blocker" is used interchangeably herein with the term "VRAC inhibitor". A VRAC blocker is a compound that inhibits the transmembrane transport of chloride or any other anion or neutral molecule in response to cell swelling or decrease in intracellular ionic strength. A compound acting as a VRAC inhibitor binds to the channel protein and exerts its inhibiting effect discernible via one or more of the methods of measuring VRAC function as described herein. Inhibiting compounds may act via one or more theoretical mode-of-actions (MoA), the main groups of which are:

1) A way to obtain inhibition is by directly obstructing (blocking) the current flow through the open channel by binding somewhere in the transmembrane pore region of the channel. Such molecules are sometimes referred to as "blockers" in the art.

2) Another - much more heterogeneous way - to obtain inhibition is by preventing or delaying the channel's natural activation processes and/or by stimulating/accelerating its natural inactivation or deactivation processes. Such molecules are expected overall to result in a lowered probability of finding an individual channel protein in its open configuration (lowered open state probability, P0). Such molecules may be mentioned collectively as "negative gating modulators", no matter which microscopic processes they influence. A negative gating modulator is also sometimes referred to as a negative allosteric modulator" or a "NAM". In WO2017198700 are explained ways to elucidate detailed MoA of a particular compound.

VRAC and VRAC inhibition is further disclosed in WO17198700.

In another embodiment, where the first anti-cancer agent comprises an UGT1A1 substrate, the agent sensitizing and/or potentiating the cancer to the first anti-cancer agent comprises an UGT1A1 inhibitor. The term "UGT1A1 inhibitor" as used herein refers to a molecule which is capable of binding to human uridine diphosphate (UDP)-glycuronosyl transferase (UGT1A1), particularly in humans or animals and decrease the enzymatic activity of the UGT1A1. UGT1A1, for example found in the liver of humans, gluronidation a range of different molecules hereinunder several anti-cancer agents, bilirubin, and other molecules usually converting them to less active and more water-soluble forms. Whether or not a particular molecule is an UGT1A1 inhibitor can be determined by examining the molecule's IC50 in vitro in a UGT1A1 inhibition assays known in the art. The term "UGT1A1 substrate" as used herein refers to a molecule which is capable of being chemically transformed by enzymatic aid of uridine diphosphate (UDP)-glycuronosyl transferase (UGT1A1). Typically, a UGT1A1 substrate is capable of being glucuronidated by UGT1A1. For example, irinotecan undergoes in the body metabolic first conversion into its active metabolite SN-38 by carboxylesterases and subsequently conversion by UGT1A1 glucuronidation into the inactive and more water-soluble SN-38-glucoronide, which is excreted from hepatic cells, e.g. via the ABCG2 efflux pump, and eliminated from the body via biliary excretion to the feces.

In a further embodiment the agent sensitizing and/or potentiating the cancer to the first anti-cancer agent is an ABCG2 inhibitor. The term "ABCG2 inhibitor" as used herein refers to a molecule which is capable of binding to the ABCG2 efflux pump in human or animal cells and thereby reduce its pumping capacity. Efflux pumps are proteinaceous transporters localized in the cytoplasmic membrane of all kinds of cells. They are active transporters, meaning that they require a source of chemical energy to perform their function.

In an still further embodiment the ABCG2 inhibitor is selected from the group consisting of Febuxostrat having CAS number 14406-53-7, Benzbromarone having CAS number 3562-84-3, Atorvastatin having CAS number 134523-0-5, Rosuvastatin having CAS number 287714-41-4, Losartan having CAS number 114798-26-4, Tacrolimus having CAS number 10498-11-3, Topiroxostat having CAS number 577778-58-6, Fenofibrate having CAS number 49562-8-9, Cyclosporine having CAS number 59865-13-3, Furosemide having CAS number 54-31-9 and Chlorothiazide having CAS number 58-94-6.

asdasd It is well known in the art how to identify modulators of ABCG2 activity for example described in Henrich et al. A High-Throughput Cell-Based Assay for Inhibitors of ABCG2 Activity" Journal of Biomolecular Screening 11(2); 2006.

In a further embodiment the ABCG2 inhibitor inhibits the efflux of the first anti-cancer agent from the cancer cell or the expression of the ABCG2 transporters compared to similar non-cancer cells.

In an alternative embodiment the agent sensitizing and/or potentiating the cancer to the first anti-cancer agent is an SRPK1 inhibitor.

Inhibitors described herein typically have an IC50 of 20 μM or less, such as 15 μM or less, such as 10 μM or less, such as 5 μM or less against the inhibitor target. In one embodiment, the IC50 is 5 μM or less, such as 4 μM or less, such as 3 μM or less, such as 2 μM or less, for example between 0.1 μM and 2.0 μM. In some embodiments, the inhibitor is non-competitive, while in other embodiments the inhibitor is competitive.

In another embodiment the agent sensitizing and/or potentiating the cancer to the first anti-cancer agent comprise a compound of general formula I:

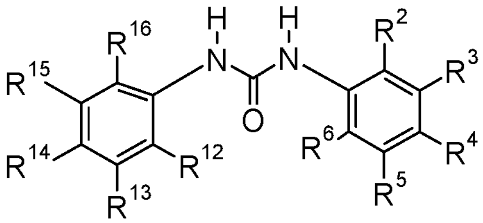

(I)

or a pharmaceutically acceptable salt thereof,

wherein R2 represents tetrazolyl; and
R3, R4, R5, R6, R12, R13, R14, R15, and R16 independently of each other represent hydrogen, halo, trifluoromethyl, nitro, alkyl, alkylcarbonyl, -NRaRb, -NRa-CO-Rb, phenyl or heteroaryl;
which phenyl is optionally substituted with halo, trifluoromethyl, nitro, -CO-NHRc, CO-O-Rc or -CO-NR'R";
wherein Rc is hydrogen, alkyl, or phenyl;
R' and R" independently of each other are hydrogen or alkyl; or
R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond; and which heterocyclic ring may optionally be substituted with alkyl;
Ra and Rb independently of each other are hydrogen or alkyl; or
R15 and R16, or R14 and R15 together with the phenyl ring to which they are attached form a naphthyl ring or an indanyl ring; and R3, R4, R5, R6, R12 and R13 and the remaining one of R14, R15 and R16 are as defined above.

In a further embodiment R3, R5, and R6 represent hydrogen; and R4 represents halo.
In a still further embodiment R3, R5, and R6 represent hydrogen; and R4 represents phenyl substituted with trifluoromethyl, nitro or -CO-NHRc; wherein Rc is phenyl.
In a still further embodiment, the sensitizing/potentiating agent is selected from the group consisting ofSCO-101, NS3623, and NS3749
In some embodiments, the sensitizing/potentiating agent comprise SCO-101 :

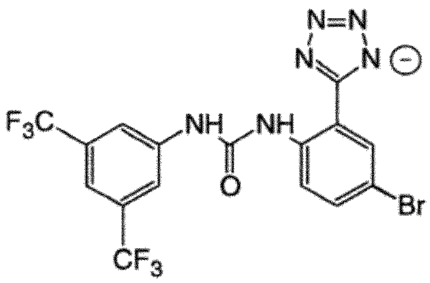

(SCO-101);

or a pharmaceutically acceptable salt thereof.
In other embodiments the sensitizing/potentiating agent inhibits one or more of UGT1A1, ABCG2 and SRPK1, optionally with an IC50 of less than 0.5.

Pharmaceutically Acceptable Salts

The sensitizing/potentiating agent as described herein may be provided in any form suitable for the intended administration. Suitable forms include pharmaceutically (i.e. physiologically) acceptable salts, and pre- or prodrug forms. Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate af derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2- sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art. Other acids such as oxalic acid, which may not be considered pharmaceutically acceptable, may be useful in the preparation of salts useful as intermediates in obtaining a chemical compound for use according to the invention and its pharmaceutically

acceptable acid addition salt. Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art. In the context of this invention the "onium salts" of N-containing compounds are also contemplated as pharmaceutically acceptable salts (aza-onium salts). Preferred azaonium salts include the alkylonium salts, in particular the methyl- and the ethylonium salts; the cycloalkylonium salts, in particular the cyclopropylonium salts; and the cycloalkylalkylonium salts, in particular the cyclopropyl-methylonium salts.

**First anti-cancer agent**

The first anti-cancer agent of the invention comprises chemotherapeutic drugs/agents. Chemotherapy drugs can be divided into groups based on factors such as how they work or their Mode-of-Action, their chemical structure, and their relationship to other drugs. Some drugs act in more than one way and may belong to more than one group. In certain embodiments the anti-cancer treatment of the present invention encompasses treatment with more than one first anti-cancer agent.

In one embodiment, the first anti-cancer agent causes neutropenia when administered to a subject in effective amounts without the sensitizing/potentiating agent, optionally grade 1, 2, 3 or 4 neutropenia or greater established by the US Cancer.

Anti-cancer agents are typically cytotoxic agents or cytostatic agents. Generally the first anti-cancer agent directly or indirectly damages the DNA, genome or genome integrity of the cancer cell.

In more specific embodiments, the first anti-cancer agent is selected from the group consisting of topoisomerase inhibitors, anti-hormone agents, alkylating agents, mitotic inhibitors, antimetabolites, anti-tumour antibiotics, corticosteroids, and differentiating agents.

Topoisomerase inhibitors

In one embodiment the first anti-cancer agent comprises a topoisomerase inhibitor may be a Topoisomerase I inhibitor or a Topoisomerase II inhibitor. These drugs interfere with enzymes called topoisomerases, which help separate the strands of DNA so they can be copied during the S phase. Topoisomerase inhibitors are typically used to treat colorectal cancer, certain leukemias, as well as lung, ovarian, gastrointestinal, and other cancers.

Topoisomerase inhibitors are grouped according to which type of enzyme they affect. Topoisomerase I inhibitors include: Topotecan, Irinotecan (CPT-11) and the active metabolite of irinotecan which is also known as SN-38.

Topoisomerase II inhibitors include: etoposide (VP-16), teniposide, mitoxantrone andanthracyclines.

In one embodiment the topoisomerase inhibitor is a Topoisomerase I inhibitor, such as Irinotecan or its active metabolite SN-38, while in another embodiment the topoisomerase inhibitor is a Topoisomerase II inhibitor, such as an anthracycline. In another embodiment, the first anti-cancer agent comprises a topoisomerase inhibitor, optionally a topoisomerase I inhibitor or topoisomerase II inhibitor, such as irinotecan or its active metabolite SN-38. In a still further embodiment, a) the first anti-cancer agent comprises a topoisomerase inhibitor, optionally a topoisomerase I inhibitor, optionally irinotecan or its active metabolite SN-38; and b) the sensitizing/potentiating agent comprises SCO-101 .

Attractive embodiments include those where a topoisomerase inhibitor, such as a topoisomerase I inhibitor, is combined with a sensitizing/potentiating agent for the treatment of colorectal cancer, in particular colorectal cancer which is resistant to treatment with said topoisomerase inhibitor. In one embodiment the colorectal cancer is metastatic colorectal cancer.

In a particular embodiment, irinotecan/SN-38 can be co-administered with a sensitizing/potentiating agent, such as SCO-101, for the treatment of colorectal cancer, in particular an irinotecan/SN-38 resistant colorectal cancer. In one embodiment the colorectal cancer is metastatic colorectal cancer.

Anti-hormone agents

The first anti-cancer agent can also comprise an anti-hormone agent. Drugs in this category are sex hormones, or hormone-like drugs, that change the action or production of female or male hormones. They are used to slow the growth of breast, prostate, and endometrial (uterine) cancers, which normally grow in response to natural sex hormones in the body. These cancer treatment hormones do not work in the same ways as standard chemotherapy drugs. They work by making the cancer cells unable to use the hormone they need to grow, or by preventing the body from making the hormone. Examples of anti-hormone agents nclude· Anti-estrogens: fulvestrant (Faslodex®), tamoxifen, and toremifene (Fareston®), clomifene, and raloxifene, anti-progestogens: mifepristone, ulipristal acetate, aglepristone, lilopristone and onapristone, Anti-androgens: bicalutamide (Casodex®), flutamide (Eulexin®), and nilutamide (Nilandron®),

Aromatase inhibitors: anastrozole (Arimidex®), exemestane (Aromasin®), and letrozole (Femara®), Progestins, megestrol acetate (Megace®), Estrogens, Gonadotropin-releasing hormone (GnRH), also known as luteinizing hormone releasing hormone (LHRH) agonists or analogs: leuprolide (Lupron®) and goserelin (Zoladex®).

In one embodiment, the anti-cancer treatment comprises anti-estrogen treatment. Anti-estrogens, also known as estrogen receptor antagonists or estrogen receptor blockers, are a class of drugs which prevent estrogens like estradiol from mediating their biological effects in the body. In another embodiment, the anti-estrogenis fulvestrant or tamoxifen.

In one embodiment, the anti-cancer treatment comprises anti-progestogen treatment. Anti-progestogens, or anti-progestins, also known as progesterone receptor antagonists or progesterone blockers, are a class of drugs which prevent progestogens like progesterone from mediating their biological effects in the body. Examples of anti-progestogens include mifepristone, ulipristal acetate, aglepristone, lilopristone and onapristone.

In one embodiment, the anti-cancer treatment comprises anti-androgen treatment. Anti-androgens, also known as androgen receptor antagonists or testosterone blockers, are a class of drugs which prevent androgens like testosterone and dihydrotestosterone (DHT) from mediating their biological effects in the body.

Anti-hormone therapy is particularly useful for treatment of steroid hormone receptor positive cancers, for example anti-estrogens are used for treatment of ER positive breast or uterine cancer.

In one embodiment, an anti-estrogen is co-administered with a sensitizing/potentiating agent, such as SCO-101 , for the treatment of an ER positive cancer, such as an ER positive breast cancer. In one embodiment the breast cancer is metastatic breast cancer.

In one embodiment, an anti-progestogen is co-administered with a sensitizing/potentiating agent, such as SCO-101, for the treatment of a PR positive cancer, such as a PR positive breast cancer. In one embodiment the breast cancer is metastatic breast cancer.

In one embodiment, an anti-androgen is co-administered with a sensitizing/potentiating agent, such as SCO-101 , for the treatment of an AR positive cancer, such as an AR positive prostate cancer. In one embodiment the prostate cancer is metastatic prostate cancer.

In one example a) the first anti-cancer agent comprises an anti-estrogen, optionally fulvestrant or tamoxifen; and b) the sensitizing/potentiating agent comprise SCO-101 .

## Alkylating agents

In some embodiments the first anti-cancer agent comprises an alkylating agent. Alkylating agents directly damage DNA (the genetic material in each cell) to keep the cell from reproducing. These drugs work in all phases of the cell cycle and are used to treat many different cancers, including glioblastoma, leukemia, lymphoma, Hodgkin disease, multiple myeloma, and sarcoma, as well as cancers of the lung, breast, and ovary.

Alkylating agents are divided into different classes, including nitrogen mustards: such as mechlorethamine (nitrogen mustard), chlorambucil, cyclophosphamide (Cytoxan®), ifosfamide, and melphalan. Alkylating agents also include Nitrosoureas: such as streptozocin, carmustine (BCNU), and lomustine, Alkyl sulfonates: busulfan, Triazines: dacarbazine (DTIC) and temozolomide (Temodar®), and/or Ethylenimines: thiotepa and altretamine (hexamethylmelamine)

In other embodiments the alkylating agent is selected from the group consisting of Nitrogen mustards, Nitrosoureas, Alkyl sulfonates, Triazines, Ethylenimine. In one embodiment the alkylating agent is a triazine, such as temozolomide. In particular the first anti-cancer agent can comprise temozolomide, and in a more particular embodiment a) the first anti-cancer agent comprises an alkylating agent, optionally temozolomide; and b) the sensitizing/potentiating agent comprises SCO-101.

Platinum drugs (such as cisplatin, carboplatin, and oxaliplatin) are sometimes grouped as alkylating agents because they kill cancer cells in a similar way. However, in som embodiment of this disclosure, platinum drugs are not considered alkylating agents. In fact in some further embodiments the first anti-cancer treatment does not comprise or consist of treatment with a metal-based anti-cancer drug, such as a platinum, ruthenium, gold or titanium- based anti-cancer drug, such as platinum-based anti-cancer drugs, including cisplatin, carboplatin, oxaliplatin or nedaplatin.

In one embodiment, the alkylating agent combined with a sensitizing/potentiating agent is used for the treatment of glioblastoma, in particular glioblastoma, which is resistant to treatment with alkylating agents. In a particular embodiment, the alkylating agent is a triazine, such temozolomide (temodal), the sensitizing/potentiating agent is SCO-101 and the cancer to be treated is glioblastoma, in particular temozolomide-resistant glioblastoma.

## Mitotic inhibitors

In one embodiment the chemotherapeutic agent comprises a mitotic inhibitor. Mitotic inhibitors are often plant alkaloids and other compounds derived from natural products. They work by stopping mitosis in the M phase of the cell

cycle but can damage cells in all phases by keeping enzymes from making proteins needed for cell reproduction. Examples of mitotic inhibitors include: Taxanes: paclitaxel (Taxol®) and docetaxel (Taxotere®), Epothilones: ixabepilone (Ixempra®), Vinca alkaloids: vinblastine (Velban®), vincristine (Oncovin®), and vinorelbine (Navelbine®), Estramustine (Emcyt®) Mitotic inhibitors are typically used to treat many different types of cancer including breast, lung, myelomas, lymphomas, and leukemias. In one embodiment the mitotic inhibitor is a taxane, such as paclitaxel or docetaxel.

In one embodiment, the first anti-cancer agent comprises a mitotic inhibitor, optionally paclitaxel or docetaxel.

In a further embodiment, a) the first anti-cancer agent comprises a mitotic inhibitor, optionally paclitaxel or docetaxel; and b) the sensitizing/potentiating agent comprise SCO-101.

In one embodiment a mitotic inhibitor is combined with the sensitizing/potentiating agent for the treatment of breast cancer, in particular breast cancer which is resistant to treatment with mitotic inhibitors. In one embodiment the breast cancer is metastatic breast cancer.

In a particular embodiment, a taxane, such as paclitaxel or docetaxel is co• administered with a VRAC modulator, such as SCO-101, for the treatment of breast cancer, in particular a paclitaxel or docetaxel resistant breast cancer. In one embodiment the breast cancer is metastatic breast cancer.

Anti-metabolites

In further embodiments the first anti-cancer agent comprises an anti-metabolite. Antimetabolites interfere with DNA and RNA growth by substituting for the normal building blocks of RNA and DNA. These agents damage cells during the S phase when the cell's chromosomes are being copied. They are commonly used to treat leukemias, cancers in the breast, ovaries and in the intestinal tract, e.g. colorectal cancer as well as other types of cancer. In one embodiment the antimetabolite is selected from the group consisting of 5-fluorouracil (5-FU), 6-mercaptopurine (6-MP), Capecitabine (Xeloda®), Cytarabine (Ara-C®), Floxuridine, Fludarabine, Gemcitabine (Gemzar®), Hydroxyurea, Methotrexate, Pemetrexed (Alimta®). In particular useful embodiments the antimetabolite is 5-fluorouracil (5-FU).

In particular embodiments a) the first anti-cancer agent comprises an antimetabolite, optionally 5-fluorouracil (5-FU); and b) the sensitizing/potentiating agent comprises SCO-101, wherein optionally the cancer is colorectal cancer, in particular a 5-FU resistant colorectal cancer, which may be a metastatic colorectal cancer.

In other embodiments the anti-metabolite is Gemcitabine and/or TAS-102.

Anti-tumor antibiotics

In further embodiments the first anti-cancer agent comprises an anti-tumor antibiotic agent, which optionally comprises an anthracycline. Anthracyclines are anti-cancer antibiotics that interfere with enzymes involved in DNA replication and works by work by altering the DNA inside cancer cells to keep them from growing and multiplying. These drugs work in all phases of the cell cycle. They are widely used for a variety of cancers. Anthracyclines are also capable of inhibiting topoisomerase II. Examples of anthracyclines include Daunorubicin, Doxorubicin (Adriamycin®), Epirubicin, Idarubicin. Anti-tumor antibiotics that are not anthracyclines include Actinomycin-D, Bleomycin, Mitomycin-C Mitoxantrone (also acts as a topoisomerase II inhibitor).

In the tumour microenvironment, immunogenic cell death (ICD) plays a major role in stimulating the dysfunctional antitumour immune system. In one embodiment, the the first anti-cancer agent increases directly or indirectly immunogenic cell death.

In another embodiment, the first anti-cancer agent directly or indirectly damages the DNA, the genome or the genome integrity of the cancer cell.

The tumor mutation burden (TMB) is a prediction of the number of mutations carried by tumor cells. Knowing the tumor mutational burden may help plan the best treatment. For example, tumors that have a high number of mutations appear to be more likely to respond to certain types of immunotherapy. In another embodiment, the first anti-cancer agent increases the Tumor mutation burden. In some embodiments the first anti-cancer agent increases the tumor mutation burden.

Corticosteroids

In further embodiments the first anti-cancer agent comprises a corticosteroid. Corticosteroids, often simply called steroids, are natural hormones and hormone-like drugs that are useful in the treatment of many types of cancer, as well as other illnesses. When these drugs are used as part of cancer treatment, they are considered chemotherapy drugs. Examples of corticosteroids include Prednisone, Methylprednisolone (Solumedrol®), and Dexamethasone (Decadron®). Steroids are also commonly used to help prevent nausea and vomiting caused by chemotherapy. They are used before chemotherapy to help prevent severe allergic reactions, too. In some embodiments the chemotherapeutic agent is not a corticosteroid.

Differentiating agents

In further embodiments the first anti-cancer agent comprises a differentiating agent. These anti-cancer drugs act on the cancer cells to make them mature into normal cells. Examples include the retinoids, tretinoin (ATRA or Atralin®) and bexarotene (Targretin®), as well as arsenic trioxide (Arsenox®).

Other chemotherapy drugs

Some anti-cancer agents act in slightly different ways and do not fit well into any of the other categories. Examples include drugs like L-asparaginase, which is an enzyme, and the proteasome inhibitor bortezomib (Velcade®).
In addition to the first anti-cancer agent the treatment can also include administration of effective amount of an additional anti-cancer agent, suitably an anti-metabolite, such as 5-fluorouracil (5-FU).

**PD-1 and/or PD-L1 inhibitor**

PD-1 inhibitors and PD-L1 inhibitors are a group of checkpoint inhibitor anti-cancer drugs that block the activity of PD-1 and PDL1 immune checkpoint proteins present on the surface of cells. PD-1 and PD-L1 inhibitors function to inhibit the association of the programmed death-ligand 1 (PD-L1) with its receptor, programmed cell death protein 1 (PD-1). The interaction of these cell surface proteins is involved in the suppression of the immune system and occurs following infection to limit the killing of bystander host cells and prevent autoimmune disease. In one embodiment, the PD-1 and/or PD-L1 inhibitor is an antibody.In another embodiment, the PD-1 and/or PD-L1 inhibitor is and monalclonal antibody.In a further embodiment, the PD-1 and/or PD-L1 inhibitor is is selected from the group consisting of Nivolumab, Pembrolizumab, Cemiplimab, Dostarlimab, Atezolizumab, Avelumab, Durvalumab, Tislelizumab, Zimberelimab, Camrelizumab, .
In one embodiment the PD-1 and/or PD-L1 inhibitor is small chemical molecule. PD-1 and/or PD-L1 inhibitor may be selected from the group consisting of Tomivosertib, BMS-1 having CAS No.: 1675201-83-8, BMS-202 having CAS No.: 1675203-84-5, PD-1-IN- having CAS No.: 1673534-76-3, BMS-1166 having CAS No.: 1818314-88-3, Fraxinellone having CAS No.: 28808-62-0, N-deacetylated BMS-202 having CAS No.: 2310135-18-1, AUNP-12 TFA, TPP-1 having CAS No.: 2426685-25-6, PD-1-IN-17 having CAS No.: 1673560-66-1.
In another embodiment the PD-1 and/or PD-L1 inhibitor is selected from the group consisting of CAS No.: 1675201-83-8, CAS No.: 1675203-84-5, CAS No.: 1673534-76-3, CAS No.: 1818314-88-3, CAS No.: 28808-62-0, CAS No.: 2310135-18-1, CAS No.: 2426685-25-6, CAS No.: 1673560-66-1.
Nivolumab is an antibody comprising a heavy chain comprising the sequence of SEQ ID NO:1 and a light chain comprising the sequence of SEQ ID NO: 2. Pembrolizumab is an antibody comprising a heavy chain comprising the sequence of SEQ ID NO: 3 and a light chain comprising the sequence of SEQ ID NO: 4. Cemiplimab is an antibody comprising a heavy chain comprising the sequence of SEQ ID NO: 5 and a light chain comprising the sequence of SEQ ID NO: 6. Dostarlimab is an antibody comprising a heavy chain comprising the sequence of SEQ ID NO:7 and a light chain comprising the sequence of SEQ ID NO: 8. Atezolizumab is an antibody comprising a heavy chain comprising the sequence of SEQ ID NO: 9 and a light chain comprising the sequence of SEQ ID NO: 10. Avelumab is an antibodycomprisingv a heavy chain comprising the sequence of SEQ ID NO:11 and a light chain comprising the sequence of SEQ ID NO: 12. Durvalumab is an antibody comprising a heavy chain comprising the sequence of SEQ ID NO:13 and a light chain comprising the sequence of SEQ ID NO: 14. Tislelizumab is an antibody comprising a heavy chain comprising the sequence of SEQ ID NO:15 and a light chain comprising the sequence of SEQ ID NO: 16. Zimberelimab is an antibody comprising a heavy chain comprising the sequence of SEQ ID NO:17 and a light chain comprising the sequence of SEQ ID NO: 18. Camrelizumab is an antibody comprising a heavy chain comprising thesequence of SEQ ID NO:19 and/or 20 and a light chain comprising the sequence of SEQ ID NO:21 and/or 22..
In one embodiment the PD-1 and/or PD-L1 inhibitor comprises an amino acid sequence which has at least 70% identity to anyone of SEQ ID NO: 1, 2, 3, 4, 5, 6, 7, 8 ,9, 10, 11, 12, 13, 14, 15, 16, 17, 18, 19, 20, 21, and/or 22, such as at least 75%, least 80%, at least 85%, at least 90%, at least 95%, at least 96%, at least 97%, at least 98%, at least 99% or at least 100% identity.

**Cancers**

The cancers encompassed herein include those cancers where the sensitizing/potentiating agent improves the effect of the first anti-cancer agent on the cancer. In some embodiments the cancer is resistant to treatment with the first anti-cancer agent. If the cancer is resistant, co-treatment with the sensitizing/potentiating agent is capable of re-sensitising the cancer to the first anti-cancer agent in question. Resistance of cancers may be either *de novo* resistance or acquired resistance. In general, a cancer is regarded as resistant to a particular first anti-cancer therapy if a patient

treated with the clinically accepted dosage of the first anti-cancer agent does not respond as expected to the first anti-cancer agent, i.e. in case of worsening, growth, or spread of the cancer (progressive disease). Whether a cancer is drug-sensitive or resistant can be determined by the skilled person using methodology known in the art.

In certain embodiments, the cancer is selected from the group consisting of adrenal cancer, acinic cell carcinoma, acoustic neuroma, acral lentigious melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatocellular carcinoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogeous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, preimary peritoneal cancer, prostate cancer, pancreatic cancer, pharyngeal cancer, pseudomyxoma periotonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's tumor, and Wilms' tumor.

In another embodiment, the cancer is selected from the group consisting of colorectal cancer, breast cancer, lung cancer (non small cell lung cancer and small cell lung cancer), glioblastomas, Head and neck cancers, malignant melanomas, basal cell skin cancer, squamous cell skin cancer, liver cancer, pancreatic cancer, prostate cancer, anal cancer, cervix uteri cancer, bladder cancer, corpus uteri cancer, ovarian cancer, gall bladder cancer, sarcomas, leukemia's (myeloid and lymphatic), lymphomas, myelomatosis, cholangiocarcinoma. colorectal cancer, breast cancer, lung cancer (non small cell lung cancer and small cell lung cancer), glioblastomas, Head and neck cancers, malignant melanomas, basal cell skin cancer, squamous cell skin cancer, liver cancer, pancreatic cancer, prostate cancer, anal cancer, cervix uteri cancer, bladder cancer, corpus uteri cancer, ovarian cancer, gall bladder cancer, sarcomas, leukemia's (myeloid and lymphatic), lymphomas, myelomatosis, cholangiocarcinoma, gastric cancer, testicular cancer, uveal cancer, mesothelioma, and merkel cell carcinoma.

In one embodiment, the cancer is a solid tumor, such as a solid tumour selected from sarcoma, carcinoma and lymphoma, while in other embodiments the cancer is not a solid tumour, such as hematological malignancy including but not limited to leukemia.

In some embodiment the cancer is metastatic, such as metastatic colorectal cancer, metastatic prostate cancer,

and/or metastatic breast cancer. In other embodiments the cancer is glioblastoma.

In a further embodiment the cancer is a steroid hormone receptor positive cancer, e.g. an estrogen receptor positive cancer, a progesterone receptor positive cancer or an androgen receptor positive cancer.

In a still further embodiment, the cancer is undifferentiated colon carcinoma cancer.

In some embodiments the cancer comprises cancer cells having an increased expression of ABCG2 compared to a corresponding non-cancer cell. In particular the cancer can comprise ABCG2+ cancer cells. ABCG2+ cancer cells for which the treatment described herein is effective are ABCG2+ cancer cells proficient in genetic mismatch repair a well as ABCG2+ cancer cell that are deficient in genetic mismatch repair. ABCG2+ are well known in the art.

Further types of cancers are described in EP1907000 and US20200323862 incoporated herein by refercence.

**Compositions and formulations**

In a further aspect also described herein is a combination of drugs for treating a cancer comprising administering to a subject in need thereof an effective amount of (i) a first anti-cancer agent, (ii) an agent sensitizing and/or potentiating the cancer to the first anti-cancer agent, and (iii) a second anti-cancer agent which comprise a PD-1 and/or PD-L1 inhibitor or pharmaceutically acceptable salts and/or prodrugs thereof. In the said combination of drugs, the first anti-cancer agent is preferably an anti-cancer agent as described herein; the sensitizing and/or potentiating agent is preferably a sensitizing and/or potentiating agent as described herein, the second anti-cancer agent is preferably a second anti-cancer agent as described herein, and the cancer is preferably a cancer as described herein.

In a separate aspect the present disclosure also describes (i) a first anti-cancer agent, suitably an anti-cancer as described herein, (ii) an agent sensitizing and/or potentiating the cancer to the first anti-cancer agent, suitably a sensitizing and/or potentiating agent described herein and (iii) a second anti-cancer agent which comprise a PD-1 and/or PD-L1, inhibitor, suitably the PD-1 and/or PD-L1 inhibitor described herein for the treatment of a disase, suitably a cancer, more suitably one or more of the cancers described herein.

The present disclosure also provides for a method for the preparation of a medicament for treating a disease, suitably a cancer, more suitably one or more of the cancers describe herein, wherein the medicament comprise (i) a first anti-cancer agent, (ii) an agent sensitizing and/or potentiating the cancer to the first anti-cancer agent, suitably the sensitizing and/or potentiating describe herein and (iii) a second anti-cancer agent which comprise a PD-1 and/or PD-L1, inhibitor, suitably the PD-1 and/or PD-L1 inhibitor described herein for the treatment of a disase, suitably a cancer, more suitably one or more of the cancers describe herein.

In another aspect the present invention relates to a pharmaceutical composition comprising an effective amount of a first anti-cancer agent and an agent sensitizing and/or potentiating the cancer to the first anti-cancer agent and a second anti-cancer agent which comprise a PD-1 and/or PD-L1 inhibitor or pharmaceutically acceptable salts and/or prodrugs thereof.

While the first anti-cancer agent and/or the agent sensitizing and/or potentiating the cancer to the first anti-cancer agent as disclosed herein may be administered in the form of the raw chemical compounds, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

The pharmaceutical composition may comprise the fist anti-cancer agent, the sensitizing and/or potentiating agent and the PD-1 and/or PD-L1 inhibitor together with one or more pharmaceutically acceptable carriers therefore, and, optionally, other therapeutic and/or prophylactic ingredients known and used in the art. The carrier(s) must be "acceptable" in the sense of being compatible with the other ingredients of the formulation and not harmful to the recipient thereof. In a further embodiment, the pharmaceutical composition comprises more than one compound or prodrug for use according to the invention, such as two different compounds or prodrugs for use according to the invention.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

In one embodiment of the present invention, the pharmaceutical composition comprises an effective amount of the sensitizing/potentiating agent, the first anti-cancer agent, the second anti-cancer agent, and one or more pharmaceutically acceptable adjuvants, excipients, carriers, buffers and/or diluents.

In another embodiment of the present invention, the composition comprises an effective amount of the sensitizing/potentiating agent, the first anti-cancer agent, and the second anti-cancer agent for use as a medicament.

In another embodiment of the present invention, the composition comprises an effective amount of the sensitizing/potentiating agent, the first anti-cancer agent, and the second anti-cancer agent for use in the treatment of cancer.

The sensitizing/potentiating agent, the first anti-cancer agent, and the second anti-cancer agent, together with a conventional adjuvant, carrier, or diluent, may thus be placed separately or together into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. The sensitizing/potentiating agent, the first anti-cancer agent, and the second anti-cancer agent as described herein can be administered separately or together in a wide variety of oral and parenteral dosage forms.

For preparing pharmaceutical compositions from a chemical compound of the present invention, pharmaceutically acceptable carriers can be either solid or liquid.

Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material. In powders, the carrier is a finely divided solid, which is in a mixture with the finely divided active component. In tablets, the active component is mixed with the carrier having the necessary binding capacity in suitable proportions and compacted in the shape and size desired. The powders and tablets suitably contain from 5 or 10 to about 70 percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration. For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify. Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution. The sensitizing/potentiating agent, the first anti-cancer agent, and the second anti-cancer agent described herein may thus be formulated separately or together for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the VRAC blocker may be in powder form, obtained by aseptic isolation of sterile solid or by lyophilization from solution, for constitution with a suitable vehicle, e.g. sterile, pyrogen-free water, before use. Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well-known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents, and the like.

For topical administration to the epidermis the sensitizing/potentiating agent, the first anti-cancer agent, and the second anti-cancer agent may be formulated separately or together as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents. Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions may be applied directly to the nasal cavity by conventional means, for example with

a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve. Alternatively, the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatine, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronisation.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these inpackaged form.

Tablets or capsules for oral administration and liquids for intravenous administration and continuous infusion are preferred compositions.

In other embodiments the sensitizing/potentiating agent, the first anti-cancer agent, and the second anti-cancer agent, are formulated separately or together for topical administration.

Further details on techniques for formulation and administration may be found in the latest edition of Remington's Pharmaceutical Sciences (Maack Publishing Co, Easton, PA).

A therapeutically effective dose refers to that amount of active ingredient, which ameliorates the symptoms or condition. Therapeutic efficacy and toxicity, e.g. ED50 and LD50, may be determined by standard pharmacological procedures in cell cultures or experimental animals. The dose ratio between therapeutic and toxic effects is the therapeutic index and may be expressed by the ratio LD50/ED50. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage of the sensitizing/potentiating agent, the first anti-cancer agent, and the second anti-cancer agent, depends on the chosen active ingredients as well as the nature and severity of the disease being treated, and is within the discretion of the physician, and may be varied by titration of the dosage to the particular circumstances to produce the desired therapeutic effect.

For example the sensitizing/potentiating agent, the first anti-cancer agent, and the second anti-cancer agent, may be administered separately or together in one or several doses per day. Exemplary ranges are from 10-200 mg/day p.o. (per oral) administered in one or two doses, such as from 25-50 mg p.o. twice a day for the sensitizing/potentiating agent.

The subject to be treated according to the present disclosure may be any human or animal subject, preferably a human suffering from cancer.

In special embodiments the sensitizing/potentiating agent, the first anti-cancer agent, and the second anti-cancer agent is suitably administered together of in any order of sequence providing optimal therapeutic efficacy. Accordingly, any sensitizing/potentiating agent, first anti-cancer agent, and second anti-cancer agent, may be administered together with, prior to or after any other sensitizing/potentiating agent, first anti-cancer agent, and second anti-cancer agent. In some embodiments administration of the sensitizing/potentiating agent, allows for administration of the first anti-cancer treatment at a lower-than-normal dose, i.e. a dose that would normally be considered a sub-therapeutic dosage.

## Kit of parts

In a separate aspect of the present invention, a kit of parts is provided, wherein the kit of parts comprises (i) a sensitizing/potentiating agent, (ii) a first anti-cancer agent and (iii) a second anti-cancer agent, wherein the second anti-cancer agent comprises a PD-1 and/or PD-L1 inhibitor and the sensitizing/potentiating agent, the first and the second anti-cancer agents are formulated for simultaneous, sequential or separate administration and optionally instructions for use.

In one embodiment of the present invention, the sensitizing/potentiating agent comprise SCO-101, the first anti-cancer agent comprises irinotecan or its active metabolite SN-38 or 5-FU and the second anti-cancer agent comprises an amino acid sequence, which has at least 70% identity to one or more sequences of SEQ ID NO: 1 or 22.

## Items of the invention

Further described herein are the following itemized embodiments:

Item 1. A method for treating a cancer comprising administering to a subject in need thereof an effective amount of (i) a first anti-cancer agent, (ii) an agent sensitizing and/or potentiating the cancer to the first anti-cancer agent, and (iii) a second anti-cancer agent which comprise a PD-1 and/or PD-L1 inhibitor or pharmaceutically acceptable salts and/or prodrugs thereof.

Item 2. The method of item 1, wherein the sensitizing/potentiating agent is co-administered with the first and/or second anti-cancer agent.

Item 3. The method of any preceding item, wherein the sensitizing/potentiating agent comprise a compound of general formula I:

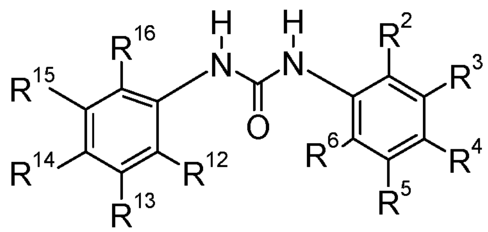

(I)

or a pharmaceutically acceptable salt thereof,

wherein R2 represents tetrazolyl; and
R3, R4, R5, R6, R12, R13, R14, R15, and R16 independently of each other represent hydrogen, halo, trifluoromethyl, nitro, alkyl, alkylcarbonyl, -NRaRb, -NRa-CO-Rb, phenyl or heteroaryl;
which phenyl is optionally substituted with halo, trifluoromethyl, nitro, -CO-NHRc, CO-O-Rc or -CO-NR'R";
wherein Rc is hydrogen, alkyl, or phenyl;
R' and R" independently of each other are hydrogen or alkyl; or
R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond; and which heterocyclic ring may optionally be substituted with alkyl;
Ra and Rb independently of each other are hydrogen or alkyl; or
R15 and R16, or R14 and R15 together with the phenyl ring to which they are attached form a naphthyl ring or an indanyl ring; and R3, R4, R5, R6, R12 and R13 and the remaining one of R14, R15 and R16 are as defined above.

Item 4. The method of item 3, wherein R3, R5, and R6 represent hydrogen; and R4 represents halo.

Item 5. The method of item 3, wherein R3, R5, and R6 represent hydrogen; and R4 represents phenyl substituted with trifluoromethyl, nitro or -CO-NHRc; wherein Rc is phenyl.

Item 6. The method of any of item 3 to 5, wherein the sensitizing/potentiating agent is selected from the group consisting of SCO-101, NS3623, and NS3749

Item 7. The method of item 6, wherein the sensitizing/potentiating agent comprise SCO-101 of the formula:

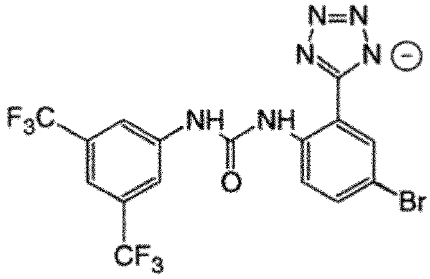

(SCO-101);

or a pharmaceutically acceptable salt thereof.

Item 8. The method of any preceding item, wherein the sensitizing/potentiating agent inhibits one or more of UGT1A1, ABCG2 and SRPK1, optionally with an IC50 of I 20 μM or less, such as 15 μM or less, such as 10 μM or less, such as 5 μM or less. In one embodiment, the IC50 is 5 μM or less, such as 4 μM or less, such as 3 μM or less, such as 2 μM or less, for example between 0.1 μM and 2.0 μM.

Item 9. The method of any preceding items, wherein the sensitizing/potentiating agent is an ABCG2 inhibitor.

Item 10. The method of any preceding items, wherein the sensitizing/potentiating agent selected from the ABCG2 inhibitors Febuxostrat, Benzbromarone, Atorvastatin, Rosuvastatin, Losartan, Tacrolimus, Topiroxostat, Fenofibrate, Cyclosporine, Furosemide, Chlorothiazide.

Item 11. The method of any preceding item, wherein the first anti-cancer agent causes neutropenia when administered to a subject in effective amounts without the sensitizing/potentiating agent.

Item 12. The method of any of item 11, wherein the neutropenia grade 3 neutropenia or greater.

Item 13. The method of any preceding item, wherein the first anti-cancer agent directly or indirectly damages the DNA and/or the genomic integrity in cancer cells.

Item 14. The method of any preceding item, wherein the first anti-cancer agent comprise a chemotherapeutic agent selected from the group consisting of topoisomerase inhibitors, anti-hormone agents, alkylating agents, mitotic inhibitors, antimetabolites, anti-tumour antibiotics, corticosteroids, and differentiating agents.

Item 15. The method of any of item 14 wherein the first anti-cancer agent comprises a topoisomerase inhibitor, optionally a topoisomerase I inhibitor or topoisomerase II inhibitor, such as irinotecan or its active metabolite SN-38.

Item 16. The method of item 15, wherein

a) the first anti-cancer agent comprises a topoisomerase inhibitor, optionally a topoisomerase I inhibitor, optionally irinotecan or its active metabolite SN-38; and
b) the sensitizing/potentiating agent comprise SCO-101.

Item 17. The method of item 14, wherein the first anti-cancer agent comprises an anti-hormone agent, optionally an anti-estrogen agent, optionally fulvestrant or tamoxifen.

Item 18. The method of item 17, wherein

a) the first anti-cancer agent comprises an anti-estrogen, optionally fulvestrant or tamoxifen; and
b) the sensitizing/potentiating agent comprise SCO-101.

Item 19. The method of item 14, wherein the the first anti-cancer agent comprises an alkylating agent, optionally temozolomide.

Item 20. The method of item 19, wherein

a) the first anti-cancer agent comprises an alkylating agent, optionally temozolomide; and

b) the sensitizing/potentiating agent comprises SCO-101.

Item 21. The method of item 14, wherein the first anti-cancer agent comprises a mitotic inhibitor, optionally paclitaxel or docetaxel.

Item 22. The method of item 21, wherein

a) the first anti-cancer agent comprise a mitotic inhibitor, optionally paclitaxel or docetaxel; and
b) the sensitizing/potentiating agent comprisess SCO-101.

Item 23. The method of item 14, wherein the first anti-cancer agent comprises an antimetabolite, optionally 5-fluorouracil (5-FU).

Item 24. The method of item 23, wherein

a) the first anti-cancer agent comprises an antimetabolite, optionally 5-fluorouracil (5-FU); and
b) the sensitizing/potentiating agent comprises SCO-101.

Item 25. The method of any preceding item, further comprising administering an effective amount of a further anti-cancer agent.

Item 26. The method of item 25, wherein the further anti-cancer agent comprises an anti-metabolite, optionally 5-fluorouracil (5-FU), Gemcitabine and/or TAS-102.

Item 27. The method of any preceding items, wherein the PD-1 and/or PD-L1inhibitor is selected from the group consisting of Nivolumab, Pembrolizumab, Cemiplimab, Dostarlimab, Atezolizumab, Avelumab, Durvalumab, Tislelizumab, Zimberelimab, Camrelizumab, and small molecules such as Tomivosertib (BMS-1 CAS No: 1675201-83-8) (BMS-202, CAS No: 1675203-84-5) (PD-1-IN-1, CAS No: 1673534-76-3) (BMS-1166 CAS No: 1818314-88-3) (Fraxinellone CAS No: 28808-62-0) (N-deacetylated BMS-202 CAS No. : 2310135-18-1), (AUNP-12 TFA (TPP-1 CAS No: 2426685-25-6) (PD-1-IN-17 CAS No: 1673560-66-1)

Item 28. The method of any preceding items, wherein the PD-1 and/or PD-L1 inhibitor comprises an amino acid sequence which has at least 70% identity to anyone of SEQ ID NO: 1 to 22.

Item 29. The method of item 28, wherein the sequence identity is at least 75%, such as at least 80%, such as at least 85%, such as at least 90%, such as at least 95%, such as at least 96%, such as at least 97%, such as at least 98%, such as at least 99%, such as 100% identity.

Item 30. The method of any preceding item, wherein the cancer is selected from the group consisting of colorectal cancer, breast cancer, lung cancer (non small cell lung cancer and small cell lung cancer), glioblastomas, Head and neck cancers, malignant melanomas, basal cell skin cancer, squamous cell skin cancer, liver cancer, pancreatic cancer, prostate cancer, anal cancer, cervix uteri cancer, bladder cancer, corpus uteri cancer, ovarian cancer, gall bladder cancer, sarcomas, leukemia's (myeloid and lymphatic), lymphomas, myelomatosis, cholangiocarcinoma, gastric cancer, testicular cancer, uveal cancer, mesothelioma, merkel cell carcinoma.

Item 31. The method of any preceding item, wherein the cancer is metastatic cancer.

Item 32. The method of item 30 to 31, wherein the cancer is colorectal cancer, such as metastatic colorectal cancer.

Item 33. The method of item 30 to 31, wherein the cancer is breast cancer, such as metastatic breast cancer.

Item 34. The method of any preceding item, wherein the cancer is resistant to the first and optionally the further anti-cancer agents.

Item 35. The method of item 34, wherein the treatment with the sensitizing/potentiating agent re-sensitises the cancer to the and optionally the further anti-cancer agents.

Item 36. The method of item 26 to 35, wherein the cancer comprises cancer cells having an increased expression

of ABCG2 compared to a corresponding non-cancer cell.

Item 37. The method of item 26 to 35, wherein the cancer comprises ABCG2+ cancer cells.

Item 38. The method of item 37, wherein the ABCG2+ cancer cell is a mismatch repair proficient ABCG2+ cancer cells.

Item 39. The method of item 37, wherein the ABCG2+ cancer cell is a mismatch repair deficient ABCG2+ cancer cells.

Item 40. A combination of drugs for treating a cancer comprising administering to a subject in need thereof an effective amount of (i) a first anti-cancer agent, (ii) an agent sensitizing and/or potentiating the cancer to the first anti-cancer agent, and (iii) a second anti-cancer agent which comprise a PD-1 and/or PD-L1 inhibitor or pharmaceutically acceptable salts and/or prodrugs thereof.

Item 41. The combination of drugs of item 40, wherein (i) the first anti-cancer agent is an anti-cancer agent of item 11 to 26; (ii) the sensitizing and/or potentiating agent is the sensitizing and/or potentiating agent of item 3 to 10, (iii) the second anti-cancer agent is the second anti-cancer agent of item 27 to 29, and (iv) the cancer is a cancer of item 30 to 39.

Item 42. A pharmaceutical composition comprising the sensitizing/potentiating agent, the first anti-cancer agent, the second anti-cancer agent, separately or together with one or more pharmaceutically acceptable adjuvants, excipients, carriers, buffers and/or diluents.

Item 43. A composition comprising separately or together effective amounts of the sensitizing/potentiating agent, the first anti-cancer agent, and the second anti-cancer agent for use as a medicament.

Item 44. A composition comprising an effective amount of amount of the sensitizing/potentiating agent, the first anti-cancer agent, and the second anti-cancer agent for use in the treatment of cancer.

Item 45. The method of any preceding item, wherein the sensitizing/potentiating agent is administered prior to and/or simultaneously with and/or after the first, the second and optionally further anti-cancer agents.

Item 46. The method of any preceding item, wherein the sensitizing/potentiating agent is administered prior to the first, the second and optionally the further anti-cancer agent.

Item 47. The method of any preceding item, wherein administration of the sensitizing/potentiating agent enhances the clinical effect of both the first and the second and optionally further anti-cancer agents.

Item 48. The method of any preceding item, wherein the the sensitizing/potentiating agent, the first, the second and optionally the further anti-cancer agent are formulated as tablets or capsules for enteral oral administration.

Item 49. The method of any preceding item, wherein the sensitizing/potentiating agent, the first, the second and optionally the further anti-cancer agents are formulated as a liquid for parenteral administration, optionally intravenous administration or continuous infusion.

Item 50. The method of any preceding item, wherein the sensitizing/potentiating agent comprises formulated as tablets or capsules for enteral oral administration, and the first, the second and optionally the further anti-cancer agents are formulated as a liquid for parenteral administration, optionally intravenous administration or continuous infusion.

Item 51. A kit of parts comprising (i) a sensitizing/potentiating agent, (ii) a first anti-cancer agent and (iii) a second anti-cancer agent, wherein the second anti-cancer agent comprises a PD-1 and/or PD-L1 inhibitor and the sensitizing/potentiating agent, the first and the second anti-cancer agents are formulated for simultaneous, sequential or separate administration and optionally instructions for use.

Item 52. The kit of parts of item 51 wherein the sensitizing/potentiating agent comprise SCO-101, the first anti-cancer agent comprises irinotecan or its active metabolite SN-38 or 5-FU and the second anti-cancer agent comprises an amino acid sequence, which has at least 70% identity to one or more sequences of SEQ ID NO: 1 to 22.

## Sequences

The present application contains a Sequence Listing prepared in PatentIn version 3.5.1, which is also submitted electronically in ST25 format which is hereby incorporated by reference in its entirety. For further reference the following sequences are included with this application:

## Examples

### Materials and methods

Materials

Chemicals used in the examples herein e.g. for buffers and substrates are commercial products of at least reagent grade.

Cancer cell line

CT26 cell line is an N-nitroso-N-methylurethane-(NNMU) induced, undifferentiated colon carcinoma cell line of BALB/c Nude mice.

Tumor cells was grown as monolayer at 37°C in a humidified atmosphere (5% CO2, 95% air). The culture medium was RPMI 1640 containing 2 mM L-glutamine supplemented with 10% fetal bovine serum. Tumor cells are adherent to plastic flasks. For experimental use, tumor cells was detached from the culture flask by a 5-minute treatment with trypsin-versene, in Hanks' medium without calcium or magnesium and neutralized by addition of complete culture medium.

Cells will be counted, and viability assessed using a 0.25% trypan blue exclusion assay.

Animals

One hundred and sixty (160) healthy female BALB/c (BALB/cByJ) mice, 6 weeks old at reception, was obtained from The Jackson Laboratory (USA).

Housing

Animals were maintained in housing rooms under controlled environmental conditions: Temperature: 20 ± 2°C, Humidity 35 ± 10%, Photoperiod (12h light/12h dark), HEPA filtered air, 15 air exchanges per hour with no recirculation. Animal enclosures provided sterile and adequate space with bedding material, food and water, environmental and social enrichment (group housing) having top filter polycarbonate Eurostandard Type III or IV cages, Corn cob bedding (ref: 7092-7097, Envigo, North America), irradiated diet 25 kGy (Envigo, North America) immunocompetent rodents (ref: 2916) 25 kGy Irradiated diet (ref: 2916, Envigo, North America), Osmotic water, and Environmental enrichment (Tube PVC ½'sterile, Canadian Tire, Canada

### Example 1 - Activity study

Animal and Cancer cell line same as above

CT26 cell line is an N-nitroso-N-methylurethane-(NNMU) induced, undifferentiated colon carcinoma cell line of BALB/c Nude mice.

Tumor induction

Tumors was induced by subcutaneous injection of $1 \times 10^6$ CT-26 cells in 200 μL of RPMI 1640 into the right flank of the 160 female animals. The day of tumor injection was considered as Day 0 (D0) of the study part.

Randomization

Animals were randomized based on their individual tumor volume. Randomization was performed when values reached a mean of 50-150 $mm^3$. 120 mice over 160 were randomized into 12 groups of 10 animals each. Homogeneity between groups were be tested by an analysis of variance (ANOVA).

Treatment

Treatment with SCO-101 was started on the day of randomization (DR). Treatment with Anti-PD1 started on the day after the randomization. Treatment with the first anti-cancer agent started two days after randomization.

The SCO-101 treatment was administered by oral gavage (PO) via a gavage tube. The recommended pH formulation for PO administration was pH 4.5 to 8.0 (min pH 2.0). The administration volume was 10 mL/kg adjusted to the most recent individual body weight.

Anti-PD-1, control isotype antibodies and Irinotecan was administered by injection into the peritoneal cavity (IP). The recommended pH formulation for IP administration was pH 7.3 - 7.4 (min 5.0 to max 9.0). The administration volume was 10 mL/kg.

5-FU was administered by intravenous injection (IV) into the caudal vein. The recommended pH formulation for IV administration was pH 5.0 - 8.0 (min pH 3.0). The administration volume was 10 mL/kg.

The treatment schedule was as follows:

Group 1 animals received *per os* administration (PO) of SCO-101 vehicle daily for five consecutive days for 3 cycles separated by two days off ((Q1Dx5)x3), combined with intraperitoneal (IP) injection of control isotype antibodies twice weekly for three consecutive weeks (TWx3) combined with intraperitoneal (IP) injection of chemo vehicle once a week for three 3 consecutive injections (Q7Dx3).

Group 2 animals received *per os* administration (PO) of SCO-101 at 75 mg/kg daily for five consecutive days for 3 cycles separated by two days off ((Q1Dx5)x3), intraperitoneal (IP) injection of control isotype antibodies twice weekly for three consecutive weeks (TWx3) combined with intraperitoneal (IP) injection of chemo vehicle once a week for three 3 consecutive injections (Q7Dx3).

Group 3 animals received *per os* administration (PO) of SCO-101 vehicle daily for five consecutive days for 3 cycles separated by two days off ((Q1Dx5)x3), intraperitoneal (IP) injection of anti-PD-1 antibody at 10 mg/kg twice weekly for three consecutive weeks (TWx3) combined with intraperitoneal (IP) injection of chemo vehicle once a week for three 3 consecutive injections (Q7Dx3).

Group 4 animals will received *per os* administration (PO) of SCO-101 vehicle daily for five consecutive days for 3 cycles separated by two days off ((Q1Dx5)x3), intraperitoneal (IP) injection of control isotype antibodies twice weekly for three consecutive weeks (TWx3) combined with intraperitoneal (IP) injection of irinotecan at 50 mg/kg once a week for three 3 consecutive injections (Q7Dx3).

Group 5 animals received *per os* administration (PO) of SCO-101 at 75 mg/kg daily for five consecutive days for 3 cycles separated by two days off ((Q1Dx5)x3), intraperitoneal (IP) injection of anti-PD-1 antibody at 10 mg/kg twice weekly for three consecutive weeks (TWx3) combined with intraperitoneal (IP) injection of chemo vehicle once a week for three 3 consecutive injections (Q7Dx3).

Group 6 animals received *per os* administration (PO) of SCO-101 at 75 mg/kg daily for five consecutive days for 3 cycles separated by two days off ((Q1Dx5)x3), intraperitoneal (IP) injection of control isotype antibodies twice weekly for three consecutive weeks (TWx3) combined with intraperitoneal (IP) injection of irinotecan at 50 mg/kg once a week for three 3 consecutive injections (Q7Dx3).

Group 7 animals received *per os* administration (PO) of SCO-101 vehicle daily for five consecutive days for 3 cycles separated by two days off ((Q1Dx5)x3), combined with intraperitoneal (IP) injection of anti-PD-1 twice weekly for three consecutive weeks (TWx3) combined with intraperitoneal (IP) injection of Irinotecan at 50 mg/kg once a week for three 3 consecutive injections (Q7Dx3).

Group 8 animals received *per os* administration (PO) of SCO-101 at 75 mg/kg daily for five consecutive days for 3 cycles separated by two days off ((Q1Dx5)x3), intraperitoneal (IP) injection of anti-PD-1 twice weekly for three consecutive weeks (TWx3) combined with intraperitoneal (IP) injection of Irinotecan at 50 mg/kg once a week for three 3 consecutive injections (Q7Dx3).

Group 9 animals received *per os* administration (PO) of SCO-101 vehicle daily for five consecutive days for 3 cycles separated by two days off ((Q1Dx5)x3), intraperitoneal (IP) injection of isotype twice weekly for three consecutive weeks (TWx3) combined with intravenous (IV) injection of 5-FU once a week for three 3 consecutive injections (Q7Dx3).

Group 10 animals received *per os* administration (PO) of SCO-101 vehicle daily for five consecutive days for 3 cycles separated by two days off ((Q1Dx5)x3), combined with intraperitoneal (IP) injection of anti-PD-1 twice weekly for three consecutive weeks (TWx3) combined with intravenous (IV) injection of 5-FU once a week for three 3 consecutive injections (Q7Dx3).

Group 11 animals received *per os* administration (PO) of SCO-101 at 75 mg/kg daily for five consecutive days for 3 cycles separated by two days off ((Q1Dx5)x3), intraperitoneal (IP) injection of isotype twice weekly for three consecutive weeks (TWx3) combined with intravenous (IV) injection of 5-FU once a week for three 3 consecutive injections (Q7Dx3).

Group 12 animals received *per os* administration (PO) of SCO-101 at 75 mg/kg daily for five consecutive days for 3 cycles separated by two days off ((Q1Dx5)x3), intraperitoneal (IP) injection of anti-PD-1 twice weekly for three consecutive weeks (TWx3) combined with intravenous (IV) injection of 5-FU once a week for three 3 consecutive injections (Q7Dx3).

The treatment schedule is summarized in the table below:

| Group | No. animals | Treatment | Dose (mg/kg/adm) | Route | Schedule |
|---|---|---|---|---|---|
| 1 | 10 | Vehicle SCO-101<br>Control Isotype<br>Vehicle chemo | -<br>10<br>- | PO<br>IP<br>IP | (Q1Dx5)x3<br>TWx3<br>Q7Dx3 |
| 2 | 10 | Vehicle SCO-101<br>Control Isotype<br>Vehicle chemo | 75<br>10<br>- | PO<br>IP<br>IP | (Q1Dx5)x3<br>TWx3<br>Q7Dx3 |
| 3 | 10 | Vehicle SCO-101<br>Control Isotype<br>Vehicle chemo | -<br>10<br>- | PO<br>IP<br>IP | ((Q1Dx5)x3<br>TWx3<br>Q7Dx3 |
| 4 | 10 | Vehicle SCO-101<br>Control Isotype<br>Irinotecan | -<br>10<br>50 | PO<br>IP<br>IP | ((Q1Dx5)x3<br>TWx3<br>Q7Dx3 |
| 5 | 10 | Vehicle SCO-101<br>Anti-PD-1<br>Vehicle chemo | 75<br>10<br>- | PO<br>IP<br>IP | ((Q1Dx5)x3<br>TWx3<br>Q7Dx3 |
| 6 | 10 | Vehicle SCO-101<br>Control Isotype<br>Irinotecan | 75<br>10<br>50 | PO<br>IP<br>IP | ((Q1Dx5)x3<br>TWx3<br>Q7Dx3 |
| 7 | 10 | Vehicle SCO-101<br>Anti-PD-1<br>Irinotecan | 10<br>50<br>- | PO<br>IP<br>IP | ((Q1Dx5)x3<br>TWx3<br>Q7Dx3 |
| 8 | 10 | SCO-101<br>Anti-PD-1<br>Irinotecan | 75<br>10<br>50 | PO<br>IP<br>IP | ((Q1Dx5)x3<br>TWx3<br>Q7Dx3 |
| 9 | 10 | Vehicle SCO-101<br>Control Isotype<br>5-FU | -<br>10<br>25 | PO<br>IP<br>IP | ((Q1Dx5)x3<br>TWx3<br>Q7Dx3 |
| 10 | 10 | Vehicle SCO-101<br>Anti-PD-1<br>5-FU | -<br>10<br>25 | PO<br>IP<br>IP | ((Q1Dx5)x3<br>TWx3<br>Q7Dx3 |
| 11 | 10 | SCO-101<br>Control Isotype<br>5-FU | 10 | PO<br>IP<br>IP | ((Q1Dx5)x3<br>TWx3<br>Q7Dx3 |
| 12 | 10 | Anti-PD-1<br>Anti-PD-1<br>5-FU | 10 | PO<br>IP<br>IP | ((Q1Dx5)x3<br>TWx3<br>Q7Dx3 |
| Total | 120 | | | | |

Clinical monitoring

All study data, including animal bodyweight measurements, tumor volume, clinical and mortality records, and

treatment was scheduled and recorded in the Vivo Manager database (Biosystemes, France). Animal viability and behavior was observed daily. A clinical follow-up was performed if deemed necessary. Body weights was measured a minimum of three times a week. The length and width of the tumor was measured a minimum of three times a week with calipers.

Health parameters

Individual, mean and median body weights of animals was measured and calculated.Mean body weight change (MBWC): average weight change of treated animals in percent (weight at day B minus weight at day A divided by weight at day A) were calculated. The intervals over which MBWC was calculated as a function of body weight curves and the days of measurement of body weight

Efficacy parameters

The treatment efficacy was assessed in terms of the effects of the test substance on the tumor volumes of treated animals relative to control animals. The following evaluation criteria of anti-tumor efficacy was determined and were chosen in consultation with the sponsor.

Tumor volume

$$\text{Tumor Volume} = \text{width}^2 \times 1/2\text{xlength}$$

Tumors which were palpable and not measurable using calipers was assigned a volume of 4 $mm^3$, indicating the technical limit measure. Tumor volume of 1000 $mm^3$ is considered to be equal to 1 g Individual, mean and median tumor volumes was provided. A comparison between group TVs may be performed at a fixed day if necessary, or in the case of MRI.

Tumor doubling time

The tumor doubling time was calculated from the day of randomization to the end of the study. For each animal, the linear regression of the points (Di, In(TV)) was calculated where Di represents the days from the randomization to the end of the study, and In(TV), the natural logarithm of the tumor volume value on the Di day. The doubling time was then calculated using the formula:

$$\frac{\ln(2)}{P}$$

Where In(2) is the natural logarithm of 2 and P the slope of the resulting regression straight. The doubling is compiled only for those animals with a correlation coefficient (slope of the linear regression) higher than the threshold of 0.70. If tumor regression is observed, the calculation of doubling is not relevant.

Tumor growth inhibition

Tumor growth inhibition (TGI) defined as the ratio of the median tumor volumes of treated versus control (T/C°/) was calculated using the formula:

$$T/C\% = 100 \times \frac{\text{Median tumor volume of treated group at DX}}{\text{Median tumor volume of vehicle treated group at DX}}$$

Where DX: Day of measurement. The optimal value is the minimal T/C% ratio reflecting the maximal tumor growth inhibition achieved. Tumor growth inhibition was classified as follows:

- <20%: high inhibition
- 21 to 40%: moderately high inhibition
- 41 to 80%: moderate inhibition
- >81%: low inhibition

Results and Conclusions:
The results are summarized in figure 1.

From the control group (G1) only 1 animal survived the tumor induction by subcutaneous injection of 1CT-26 cells.

Treatment with SCO-101 alone (G2) did not have any effect on the survival rate.

Treatment with the anti-PD-1 antibody alone (G3) exhibited a significant effet and 6 out of 10 animals survived the tumor induction.

Treatment with irinotecan alone (G4) did not have any effect on the survival rate since no animals survid the the tumor induction.

Treatment with SCO-101 in combination with the anti-PD-1 antibody (G5) did not improve the survival rate. Thus, SCO-101 does not appear to sensitize and/or potentiate the cancer cells to the anti-cancer treatment effect of the D-1 and/or PD-L1 inhibitor.Treatment with SCO-101 in combination irinotecan (G6) did show a significant effet of the ability of SCO-101 to sensitize and/or potentiate the effect of irinotecan for sensitizing cancer cells to an anti-cancer treatment.

Treatment with the anti-PD-1 antibody in combination with irinotecan (G7) resulted in a small but significant improvement in the survival rate of the animals as 8 out of 10 animals survived.

Treatment with the with SCO-101 in combination with both irinotecan and the anti-PD-1 antibody (G8) resulted in the survival of all the animals, suggesting that SCO-101 sensitize and/or potentiate the cancer cells to the anti-cancer treatment and thereby promoting irinotecan and the anti-PD-1 antibody to act synergistically.

Treatment with the with 5-FU alone (G9) resulted in the survival of 2 animals.

Treatment with the with 5-FU in combination with the anti-PD-1 antibody (G10) resulted in the survival of 7 animals.

Treatment with SCO-101 in combination 5-FU (G11) showed a small effet of the ability of SCO-101 to sensitize and/or potentiate the effect of 5-FU for sensitizing cancer cells to an anti-cancer treatment as animals survived the tumor induction.

Treatment with SCO-101 in combination with 5-FU and the anti-PD-1 antibody (G12) resulted in the survival of 9 out of 10 animals, suggesting that SCO-101 sensitize and/or potentiate the cancer cells to the anti-cancer treatment and thereby promoting 5-FU and the anti-PD-1 antibody to act synergistically which was also observed for the similar irinotecan experiment (G8).

## Claims

1. A combination of drugs for the treatment of cancer comprising (i) irinotecan or its active metabolite SN-38, (ii) SCO-101 of the formula (i):

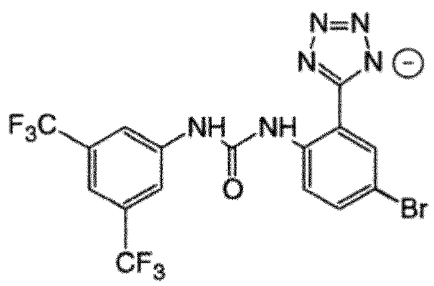

(I)

or a pharmaceutically acceptable salt thereof, and (iii) a second anti-cancer agent which comprise a PD-1 and/or PD-L1 inhibitor or a pharmaceutically acceptable salt and/or prodrug thereof.

2. The combination of drugs of claim 1, further comprising a further anti-cancer agent.

3. The comination of claim 2, wherein the further anti-cancer agent comprises an anti-metabolite, optionally 5-fluorouracil (5-FU), Gemcitabie or TAS-102.

4. The combination of drugs of any preceding claims, wherein the PD-1 and/or PD-L1 inhibitor is selected from the group consisting of Nivolumab, Pembrolizumab, Cemiplimab, Dostarlimab, Atezolizumab, Avelumab, Durvalumab, Tislelizumab, Zimberelimab, Camrelizumab, Sym021, (SG001 similar to Nivolumab) and small molecules such as Tomivosertib (BMS-1 CAS No: 1675201-83-8) (BMS-202, CAS No: 1675203-84-5) (PD-1-IN-1, CAS No: 1673534-76-3) (BMS-1166 CAS No: 1818314-88-3) (Fraxinellone CAS No: 28808-62-0) (N-deacetylated BMS-202 CAS No: 2310135-18-1), (AUNP-12 TFA (TPP-1 CAS No. : 2426685-25-6) (PD-1-IN-17 CAS No: 1673560-66-1).

5. The combination of drugs of any preceding claims, wherein the PD-1 and/or PD-L1 inhibitor comprises an amino acid sequence which has at least 90% identity to anyone of SEQ ID NO: 1 to 22.

6. The combination of drugs of claim 5, wherein the sequence identity is at least 95%, such as at least 96%, such as at least 97%, such as at least 98%, such as at least 99%, such as 100% identity.

7. The combination of drugs of any of the preceding claims, wherein the cancer is selected from the group consisting of colorectal cancer, breast cancer, lung cancer (non small cell lung cancer and small cell lung cancer), glioblastomas, head and neck cancers, malignant melanomas, basal cell skin cancer, squamous cell skin cancer, liver cancer, pancreatic cancer, prostate cancer, anal cancer, cervix uteri cancer, bladder cancer, corpus uteri cancer, ovarian cancer, gall bladder cancer, sarcomas, leukemia's (myeloid and lymphatic), lymphomas, myelomatosis, cholangiocarcinoma, gastric cancer, testicular cancer, uveal cancer, mesothelioma, merkel cell carcinoma.

8. The combination of drugs of any of the preceding claims, wherein the cancer is metastatic cancer.

9. The combination of drugs of claim 7 to 8, wherein the cancer is colorectal cancer, such as metastatic colorectal cancer.

10. The combination of drugs of claim 7 to 8, wherein the cancer is breast cancer, such as metastatic breast cancer.

11. The combination of drugs of any of the preceding claims, wherein the cancer is resistant to the first and optionally the further anti-cancer agent.

12. The combination of drugs of claim 11, wherein the SCO-101 re-sensitises the cancer to the first anti-cancer agent and optionally the further anti-cancer agents.

13. The combination of drugs of any of the preceding claim, wherein the SCO-101 is administered prior to and/or simultaneously with and/or after the first, the second and optionally the further anti-cancer agent.

14. The combination of drugs of any of the preceding claims, wherein the SCO-101 is administered prior to the first, the second and optionally the further anti-cancer agent.

15. The combination of drugs of any of the preceding claims, wherein administration of the SCO-101 enhances the therapeutical effect of both the first and the second and optionally the further anti-cancer agent.

16. The combination of drugs of any of the preceding claims, wherein (i) the SCO-101 is formulated as a tablet or a capsule for enteral oral administration, (ii) the first and/or the further anti-cancer agent is formulated as a tablet or a capsule for enteral oral administration or as a liquid for parenteral administration, optionally intravenous administration or continuous infusion, and (iii) the second anti-cancer agent is formulated as a liquid for parenteral administration, optionally intravenous administration or continuous infusion.

17. A pharmaceutical composition comprising the combination of drugs of any preceeding claim, and one or more pharmaceutically acceptable adjuvants, excipients, carriers, buffers and/or diluents.

# Figures

**Figure 1**

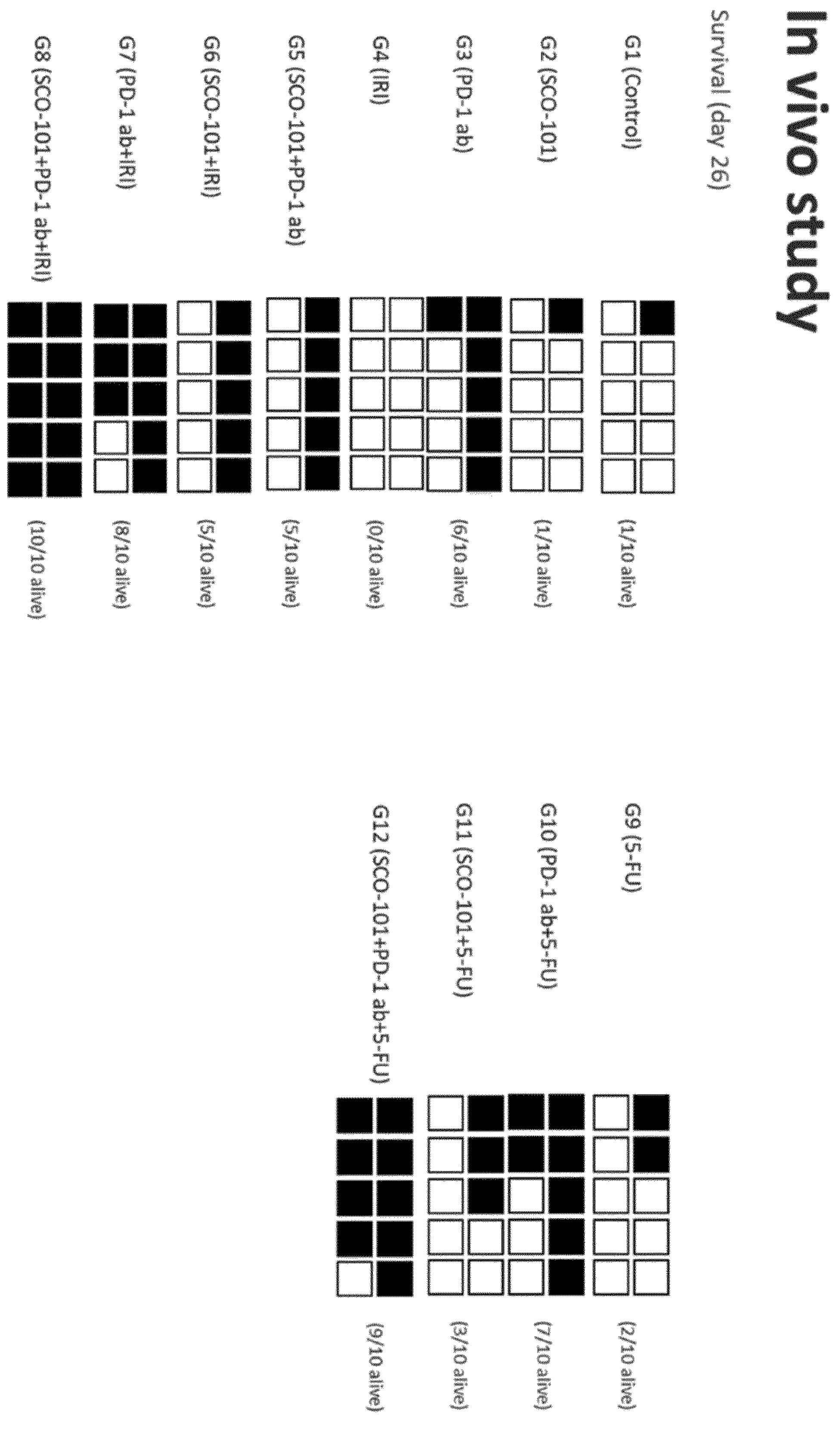

Figure 2

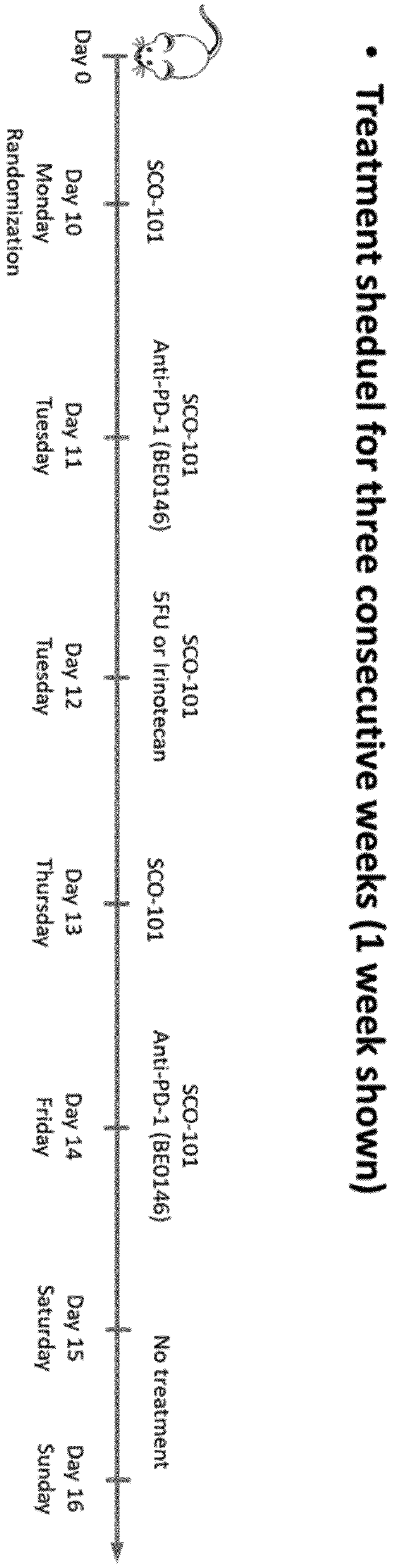

• Treatment sheduel for three consecutive weeks (1 week shown)
Day 0
SCO-101
Day 10
Monday
Randomization
SCO-101
Anti-PD-1 (BE0146)
Day 11
Tuesday
SCO-101
5FU or Irinotecan
Day 12
Tuesday
SCO-101
Day 13
Thursday
SCO-101
Anti-PD-1 (BE0146)
Day 14
Friday
No treatment
Day 15
Saturday
Day 16
Sunday

Europäisches Patentamt
European Patent Office
Office européen des brevets

# EUROPEAN SEARCH REPORT

Application Number
EP 21 21 6181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/198700 A1 (SANIONA AS [DK]) 23 November 2017 (2017-11-23)<br>* page 31, line 18 - line 21 *<br>* page 27, line 7 - line 9 *<br>* page 36, line 21 - line 22 *<br>* page 29, line 15 - line 19 *<br>* page 29, line 20 *<br>* claim 1 *<br>* page 27, line 10 - line 14 *<br>----- | 1-3, 7-15,17 | INV.<br>A61K9/00<br>A61K31/41<br>A61K31/4745<br>A61K39/00<br>A61K45/06<br>A61P35/00<br>A61P35/04 |
| X | WO 2020/049139 A1 (SCANDION ONCOLOGY AS [DK]) 12 March 2020 (2020-03-12)<br>* claim 29 *<br>* claims 11,12 *<br>* page 21, line 28 - line 29 *<br>* claim 31 *<br>* page 14, line 16 *<br>* page 21, line 1 - line 3 *<br>* page 9, line 8 - line 10 *<br>----- | 1-17 | |
| A | JAN STENVANG ET AL: "The volume regulated anion channel inhibitor NS3728 to enhance the cytotoxic effects of SN-38 in human colorectal cancer cells grown in vitro", JOURNAL OF CLINICAL ONCOLOGY, AMERICAN SOCIETY OF CLINICAL ONCOLOGY, US, vol. 34, no. 15, Suppl, 1 May 2016 (2016-05-01), page e23170, XP009195160, ISSN: 0732-183X, DOI: 10.1200/JCO.2016.34.15_SUPPL.E23170<br>* abstract *<br>-----<br>-/-- | 1-17 | TECHNICAL FIELDS SEARCHED (IPC)<br>A61K<br>A61P |

The present search report has been drawn up for all claims

1

| Place of search | Date of completion of the search | Examiner |
|---|---|---|
| Munich | 1 June 2022 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 21 6181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROGERS JANE E. ET AL: "Nivolumab in Combination with Irinotecan and 5-Fluorouracil (FOLFIRI) for Refractory Advanced Gastroesophageal Cancer", ONCOLOGY : INTERNATIONAL JOURNAL OF CANCER RESEARCH AND TREATMENT, vol. 98, no. 5, 1 January 2020 (2020-01-01), pages 289-294, XP055924996, CH ISSN: 0030-2414, DOI: 10.1159/000505974 Retrieved from the Internet: URL:https://www.karger.com/Article/Pdf/505974> * abstract * ----- | 1-17 | |
| A | SONG YAN ET AL: "HX008, an anti-PD1 antibody, plus irinotecan as second-line treatment for advanced gastric or gastroesophageal junction cancer: a multicenter, single-arm phase II trial", JOURNAL FOR IMMUNOTHERAPY OF CANCER, vol. 8, no. 2, 1 October 2020 (2020-10-01), page e001279, XP055924994, DOI: 10.1136/jitc-2020-001279 Retrieved from the Internet: URL:https://jitc.bmj.com/content/jitc/8/2/e001279.full.pdf?with-ds=yes> * abstract * ----- | 1-17 | TECHNICAL FIELDS SEARCHED (IPC) |
| | -/-- | | |

The present search report has been drawn up for all claims

1

| Place of search | Date of completion of the search | Examiner |
|---|---|---|
| Munich | 1 June 2022 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 21 6181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Hagen Jakob ET AL: "85 Poster Session Evaluation of SN-38 PK profile in patients with RAS wild-type metastatic colorectal cancer treated with a combination of SCO-101 and FOLFIRI", , 19 January 2022 (2022-01-19), pages 185-185, XP055925030, Retrieved from the Internet: URL:https://d32wbias3z7pxg.cloudfront.net/meeting/286/abstract/pdf/286-14170-204634.pdf [retrieved on 2022-05-25] * the whole document * ----- | 1-17 | |

TECHNICAL FIELDS SEARCHED (IPC)

The present search report has been drawn up for all claims

1

| Place of search | Date of completion of the search | Examiner |
|---|---|---|
| Munich | 1 June 2022 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 21 6181

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2022

| Patent document cited in search report | Publication date | Patent family member(s) | Publication date |
|---|---|---|---|
| WO 2017198700 A1 | 23-11-2017 | AU 2017266724 A1 | 20-12-2018 |
| | | BR 112018073518 A2 | 19-03-2019 |
| | | CA 3023202 A1 | 23-11-2017 |
| | | CN 109475535 A | 15-03-2019 |
| | | DK 3458052 T3 | 03-02-2020 |
| | | DK 3622953 T3 | 26-04-2021 |
| | | EP 3458052 A1 | 27-03-2019 |
| | | EP 3622953 A1 | 18-03-2020 |
| | | EP 3854393 A1 | 28-07-2021 |
| | | ES 2770374 T3 | 01-07-2020 |
| | | ES 2870805 T3 | 27-10-2021 |
| | | HR P20200076 T1 | 03-04-2020 |
| | | HR P20210572 T1 | 14-05-2021 |
| | | HU E047542 T2 | 28-04-2020 |
| | | HU E053744 T2 | 28-07-2021 |
| | | JP 7033554 B2 | 10-03-2022 |
| | | JP 2019519506 A | 11-07-2019 |
| | | LT 3458052 T | 10-02-2020 |
| | | LT 3622953 T | 26-04-2021 |
| | | PL 3458052 T3 | 29-06-2020 |
| | | PL 3622953 T3 | 16-08-2021 |
| | | PT 3458052 T | 03-02-2020 |
| | | PT 3622953 T | 21-04-2021 |
| | | RS 61786 B1 | 30-06-2021 |
| | | SI 3458052 T1 | 30-04-2020 |
| | | SI 3622953 T1 | 31-05-2021 |
| | | US 2019269654 A1 | 05-09-2019 |
| | | US 2021353596 A1 | 18-11-2021 |
| | | WO 2017198700 A1 | 23-11-2017 |
| WO 2020049139 A1 | 12-03-2020 | EP 3846802 A1 | 14-07-2021 |
| | | US 2021251963 A1 | 19-08-2021 |
| | | WO 2020049139 A1 | 12-03-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 17198700 A
- WO 2017198700 A
- EP 1907000 A
- US 20200323862 A


### Non-patent literature cited in the description


- *CHEMICAL ABSTRACTS,* 14406-53-7
- *CHEMICAL ABSTRACTS,* 3562-84-3
- *CHEMICAL ABSTRACTS,* 134523-0-5
- *CHEMICAL ABSTRACTS,* 287714-41-4
- *CHEMICAL ABSTRACTS,* 114798-26-4
- *CHEMICAL ABSTRACTS,* 10498-11-3
- *CHEMICAL ABSTRACTS,* 577778-58-6
- *CHEMICAL ABSTRACTS,* 49562-8-9
- *CHEMICAL ABSTRACTS,* 59865-13-3
- *CHEMICAL ABSTRACTS,* 54-31-9
- *CHEMICAL ABSTRACTS,* 58-94-6
- **HENRICH et al.** A High-Throughput Cell-Based Assay for Inhibitors of ABCG2 Activity. *Journal of Biomolecular Screening,* 2006, vol. 11 (2
- *CHEMICAL ABSTRACTS,* 1675201-83-8
- *CHEMICAL ABSTRACTS,* 1675203-84-5
- *CHEMICAL ABSTRACTS,* 1673534-76-3
- *CHEMICAL ABSTRACTS,* 1818314-88-3
- *CHEMICAL ABSTRACTS,* 28808-62-0
- *CHEMICAL ABSTRACTS,* 2310135-18-1
- *CHEMICAL ABSTRACTS,* 2426685-25-6
- *CHEMICAL ABSTRACTS,* 1673560-66-1
- Remington's Pharmaceutical Sciences. Maack Publishing Co